# EUROPEAN PATENT APPLICATION

(11) **EP 0 521 348 A1**
(43) Date of publication of application: **07.01.1993**
(21) Application number: 92110262.0
(22) Date of filing: 17.06.1992
(51) Int. Cl.: C07K 15/00, C12N 15/36, C12P 21/00, C12P 21/08, C12N 5/06

(54) **Chimeric antibody with specificity for hepatitis B virus pre-S2 surface antigen and cell line producing same**

(30) Priority: 18.06.1991 KR 1004091; 06.02.1992 KR 169292; 04.05.1992 KR 759392
(71) Applicant: KOREA INSTITUTE OF SCIENCE AND TECHNOLOGY, Seoul (KR)
(72) Inventor: Han, Moon Hi, Dong-ku, Daejeon (KR); Hong, Hyo Jeong, Yuseong-ku, Daejeon (KR); Ryu, Chun Jeih, Yuseong-ku, Daejeon (KR); Park, Sung Sup, Seo-ku, Daejeon (KR); Jin, Byung Rae, Kwonsun-ku, Suwon, Kyungki-do (KR); Chung, Hong Keun, Seocho-ku, Seoul (KR)
(74) Representative: Turi, Michael, Dipl.-Phys.

(57) **Abstract**

The present invention primarily pertains to a murine/human chimeric antibody specific for HBV pre-S2 antigen useful for the development of an agent for the prevention and/or treatment of hepatitis B.

The present invention further relates to a first gene encoding the heavy chain of the chimeric antibody which comprises a polynucleotide encoding the V region of the heavy chain of a murine antibody with specificity for HBV pre-S2 antigen and the C region of the heavy chain of human immunoglobulin, and a second gene encoding the kappa chain of the chimeric antibody which comprises a polynucleotide encoding the V region of the kappa chain of the murine antibody and the C region of the kappa chain of human immunoglobulin; to expression vectors which are capable of expressing the chimeric gene encoding the chimeric antibody in a compatible host cell; and to cell lines transfected with the vectors and producing the chimeric antibody.

Another aspect of the present invention is to provide a method producing the chimeric antibody by employing the cell line.

## Description

### Field of the Invention

The present invention relates to a polynucleotide encoding a murine/human chimeric antibody with specificity for hepatitis B virus pre-S2 surface antigen; to a vector capable of expressing the polynucleotide; to a cell line producing the antibody; and to the antibody produced therefrom.

### Background of the Invention

Hepatitis B caused by hepatitis B virus(HBV) has been an serious public health problem throughout the world affecting more then 300 million peple, about 75% of whom live in Asiatic countries (Tiollais and Buendia, April 1991, Scientific American). HBV may be classified into subtypes in accordance with the serological classification of people; and, in the East Asia, HBV adr subtype is known to be predominant(Couroucé-Pauty et al., Vox Sang 44, 197-211(1983)).

Further studies on HBV have uncovered that serveral antigens, e.g., S, pre-S1 and pre-S2, exist on the surface of HBV; and, further, that pre-S2 antigen consisting of 55 amino acids contains a strong epitope(Milich et al., Science 228, 1195-1199(1985)) and induces the production of a neutralizing antibody against HBV (Neurath et al., Vaccine 4, 35-37(1986)).

A common conventional method to prevent or treat hepatitis B is to administer anti-hepatitis B immune globulin(HBIG) to actual or potential patients; and such immunoglobulin is normally derived from pooled human anti-hepatitis B surface antigen(anti-HBsAg) positive plasma. The potential risk stemming from the infectious agents and diminishing sources that may be present in the pool, however, tend to limit the clinical use of such HBIG derived from human plasma for passive immunization. Consequently, monoclonal antibodies against HBsAg have been tried to overcome the drawback (Iwarson et al., J. Med. Virol. 16, 89-96(1985)).

However, most monoclonal antibodies are of murine origin and may have limited utility in human therapy because they usually elicit an immune response in patients(Miller et al., Blood 62, 988-995(1983); Shawler et al., J. Immunol. 135, 1530-1535(1985)). Such response reduces therapeutic efficacy and may also entail undesirable clinical side effects. Human monoclonal antibodies would circumvent this problem; but they are difficult to produce and often have low affinity to antigens(Carson and Freimark, Adv. Immun. 38, 275-310(1986)).

To solve the above problems, therefore, chimeric antibodies, consisting of a murine variable(V) region which operates to bind to antigens and a human constant(C) region which plays the role of an effector, represent a compromise which may retain the affinity and specificity of the parental murine monoclonal antibodies and eliminate the patient immune response to the murine C region(Morrison et al., Proc. Natl. Acad. Sci. U.S.A. 81, 6851-6855(1984); Sahagan et al., J. Immunol. 137, 1066-1074(1986)).

Further, humanized antibodies wherein complementarity determining regions(CDRs) of the human V region are replaced with those of murine have been developed(Riechmann et al., Nature 332, 323-327(1988); Queen et al., Proc. Natl. Acad. Sci. U.S.A. 86, 10029-10033(1989); Tempest et al., Bio/Technology 9, 266-271 (1991)).

A human monoclonal antibody and a murine/human chimeric antibody with specificity for S antigen, one of the HBV surface antigens, have been reported by Harada et al. in Bio/Technology 7, 374-377, and by Li et al. in Molecular Immunology 27, 303-311 (1990), respectively. Pre-S2 antigen, another HBV suface antigen, which is protruded on the surface of HBV is reported to have enhanced immunogenicity, as compared with S antigen(Milich et al., Science 228, 1195-1199(1985)); accordingly, it could be postulated that an antibody specific for pre-S2 antigen together with an antibody speccific for S antigen is useful for developing an improved agent for prevention and treatment of hepatitis B. However, successful isolation of a gene encoding an antibody specific for HBV pre-S2 antigen as well as a chimeric antibody against pre-S2 antigen has not yet been reported.

### Summary of the Invention

Accordingly, the present invention primarily pertains to a murine/human chimeric antibody specific for HBV pre-S2 antigen useful for the development of an agent for the prevention and/or treatment of hepatitis B.

The present invention is further directed to a first gene encoding the heavy chain of a chimeric antibody which comprises a polynucleotide encoding the V region of the heavy chain of a murine antibody with specificity for HBV pre-S2 antigen and the C region of the r1 heavy chain of human immunoglobulin, and a second gene encoding the kappa chain of the chimeric antibody which comprises a polynucleotide encoding the V region of the kappa chain of the murine antibody and the C region of the kappa chain of human immunoglobulin; to expresssion vectors which are capable of expressing the chimeric gene encoding the chimeric antibody in a compatible host cell; and to cell lines transfected with the vectors.

Another aspect of the present invention is to provide a method for producing the chimeric antibody by employing the cell line.

### Brief Description of the Drawings

The invention can be more readily understood by reference to the accompanying drawings in which:
- Fig. 1A: shows the restriction map of the gene encoding the V region of the heavy chain of the murine antibody specific for the HBV pre-S2 antigen, and Fig. 1B shows the nucleotide sequence of a part of said gene and the amino acid sequence deduced therefrom.
- Fig. 2A: shows the restriction map of the gene encoding the kappa chain of the murine antibody, and Fig. 2B shows the nucleotide sequence of a part of said gene and the amino acid sequence deduced therefrom.
- Fig. 3: shows the strategy for the construction of a recombinant plasmid pSV2neo-hCrl which contains a gene encoding the C region of the heavy chain of human immunoglobulin G.
- Fig. 4: shows the strategy for the construction of pHS2neo which is an expression vector for the gene encoding the heavy chain of the chimeric antibody specific for the HBV pre-S2 antigen.
- Fig. 5: shows the strategy for the construction of pLS2-hygro which is an expression vector for the gene encoding the kappa chain of the chimeric antibody specific for HBV pre-S2 antigen.
- Fig. 6: shows the result of ELISA(enzyme linked immunosorbent assay) for the culture medium after the incubation of a murine myeloma cell which was transfected with expression vectors pHS2-neo and pLS2-hygro.
- Fig. 7A: shows the results of SDS-PAGE of the chimeric antibody isolated from the culture of transfectoma cells A-44 and 6-31.
- Fig. 7B: shows the results of western blotting analyses with the gel of Fig. 7A.

### Detailed Description of the Invention

All references cited herein are hereby incorporated in their entirety by reference.

As used herein, the following terms shall have the following meanings:

The term "chimeric antibody" refers to an antibody composed of parts originated from more than one cell which are genetically different.

The term "immunoglobulin" refers to an antibody secreted by mature lymphoid cells called plasma cells, which is Y-shaped and has tetrameric molecules consisting of two relatively long polypeptide chains; in the case of immunoglobulin G, which is the predominant molecule involved in the secondary immune responses, each of the chains consists of a variable(V) region and a constant (C) region.

The term "expression vector" refers to a cloning vehicle designed to promote the expression of polynucleotide inserts.

The other terms used herein have their normal and conventional meanings as used in the art. The present invention will be more specifically illustrated below.

The gene encoding a murine/human chimeric antibody specific for HBV pre-S2 antigen of the present invention may be constructed as follows:
First, a genomic DNA is isolated from a hybridoma cell line which produces a murine monoclonal antibody specific for HBV pre-S2 antigen, for example, H8 hybridoma cell line(Chung and Kim, Recent Progress in Molecular Biology and Genetic Engineering in Korea 1, 165-172(1987)) producing a murine monoclonal antibody specific for pre-S2 antigen of HBV adr subtype.

Secondly, for the cloning of each of the genes encoding the V region of the heavy chain(V_{H} gene) and the kappa chain(V_{K} gene), the isolated genomic DNA is partially or completely digested with (an) appropriate restriction endonuclease(s); the digested DNA fragments are inserted into λ phage DNA or plasmid DNA; and, the recombinant λ DNA or plasmid DNA is introduced into E. coli cells and propagated to obtain a DNA library.

Thirdly, the genomic library is plated on E. coli cells to form phage plaques, which are, then, screened by plaque hybridization with (an) appropriate probe(s) to select phage clones to which the probe(s) binds complementarily; positive plaques are isolated and analysed; and, the desired genes may be obtained therefrom(Maniatis et al., 1989, Molecular Cloning: A Laboratory Manual, CSH Lab., N.Y., U.S.A.). Alternatively, the genomic library may be prepared by employing a plasmid, instead of λ phage, in which case the colony hybridization method is used for the screening of positive colonies(Maniatis et al., 1989, vide supra).

The nucleotide sequence of the cloned genes may be determined by employing, e.g., the dideoxy chain-termination method (Sanger, Proc. Natl. Acad. Sci. U.S.A., 74, 5463(1977)). From the nucleotide sequence analysis, it can be confirmed whether each of the genes contains a functionally rearranged V_{H} gene or V_{K} gene and transcriptional elements which are required for B cell-specific expression.

Thereafter, each of the V_{H} and V_{K} genes is connected with the gene encoding the C region of the r1 heavy chain of human immunoglobulin(C_{H} gene) and with the gene encoding the C region of the kappa chain of human immunoglobulin(C_{K} gene), respectively, for the purpose of obtaining the genes encoding the murine/human chimeric kappa chain and heavy chain. Each of the genes is cloned into an expression vector; and microbial host cells are transformed therewith.

A non-immunoglobulin secreting murine hybridoma cell line is co-transfected with both of the expression vectors isolated from the cultured transformants wherein the cell line is, first, transfected with one of the expression vectors and selected in a medium containing a first drug which can be used to select the cells expressing the vector; and, then, the selected cells are transfected again with the other expression vector and selected in a medium containing a second drug which can select the cells expressing the other vector.

The co-transfected cells which are alive through the two selection procedures are cultured in each well of a microplate; and the existence and the level of the chimeric antibody produced from each of the transfectoma cells may be determined by using a conventional method such as ELISA.

For example, ELISA may be carried out as follows:
First, the culture of the transfectoma cells are lysed and centrifuged; and the supernatant is distributed in the wells of a microtiter plate to make antibodies, if any, adsorb onto the surface of the well;
Secondly, bovine serum albumin is added to the well for the purpose of blocking the protein-binding site of the well;
Thirdly, an enzyme, e.g., HRP(horseradish peroxidase) conjugated anti-human Immunoglobulin G is added to the well to allow the anti-human kappa chain-HRP to bind to the antibodies of the first step; and
Finally, a substrate for the enzyme, e.g., ABTS^{R} (Boeringermanheim Co., F.R.G.) and hydrogen peroxide for peroxidase are added to the well to develop a color reaction.

When the supernatant contains antibodies, color appears as a result of the reaction of the enzyme with the substrate. The color reaction is stopped by addition of diluted sulfuric acid.

The degree of color intensity can be measured with a microwell reader; and the existence and the level of antibodies can be determined therefrom.

The chimeric antibodies produced from the transfectoma cells may be easily isolated and purified by employing a conventional method such as affinity chromatography.

Although the above illustration of the present invention has been made with respect to the kappa chain alone as the light chain for constructing an antibody, it should be understood that other types of light chains such as the lambda chain may also be employed since the kappa chain may be substituted with the lambda chain without limiting the functionality of an antibody.

The polynucleotides of this invention can also be isolated and cloned from a genomic library, or can be chemically synthesized by using a suitable method such as the phosphoamidite solid support method proposed by Matteuchi et al.(J. Am. Chem. Soc. 103, 3185 (1981)).

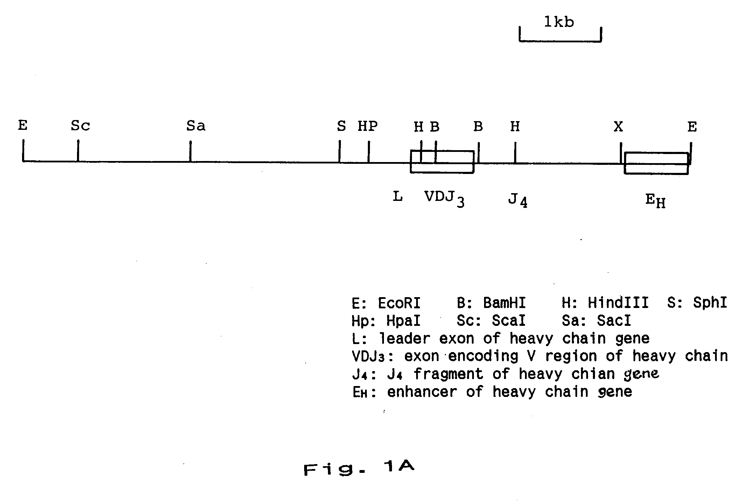

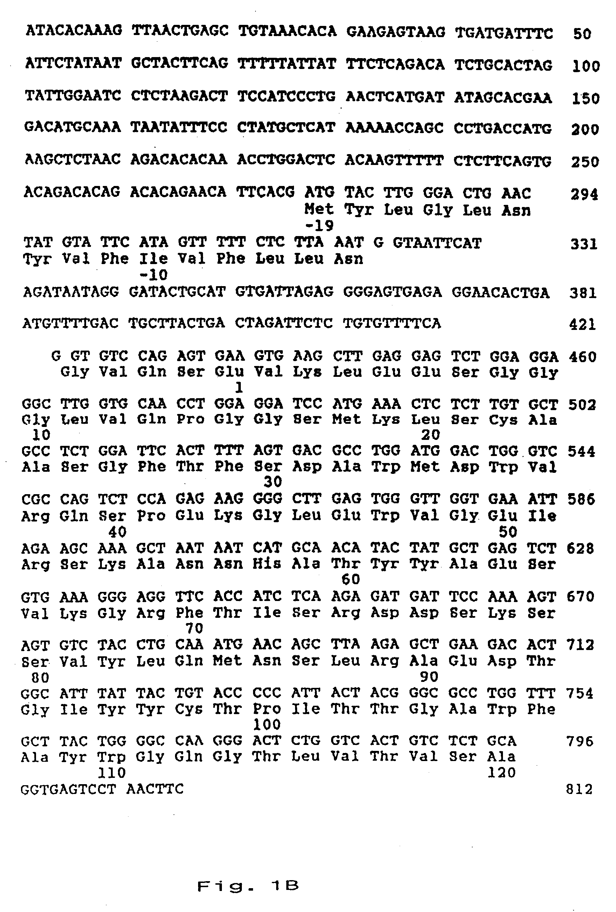

Further, because of the degeneracy of the genetic code, it will be understood that there are many potential nucleotide sequences that could code for, e.g., the amino acid sequence of Figs. 1B and 2B.

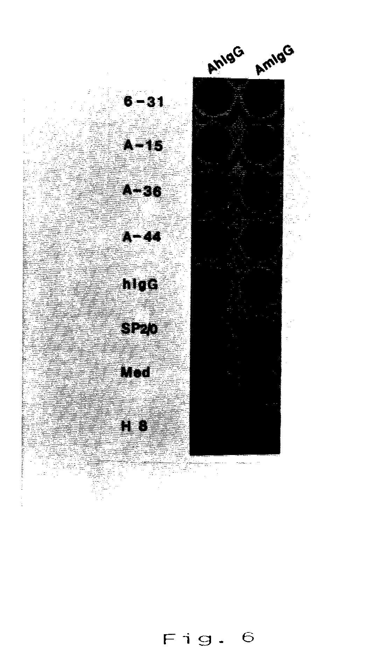

On the other hand, amino acid substitutions in proteins which do not substantially alter biological and immunological activities have been known to occur and have been described, e.g., by Neurath et al. in "The Proteins", Academic Press, New York(1979), and, in particular, in Fig. 6 appeaing on page 14 thereof. Most frequently observed amino acid substitutions are Ala/Ser, Val/Ile, Asp/Glu, Thr/Ser, Ala/Gly, Ala/Thr, Ser/Asn, Ala/Val, Ser/Gly, Thr/Phe, Ala/Pro, Lys/Arg, Asp/Asn, Leu/Ile, Leu/Val, Ala/Glu, Asp/Gly, and vice versa.

Such functionally equivalent amino acid substitutions of the exemplary embodiments of this invention are within the scope of the invention as long as the resulting proteins retain the characteristic of the antibody, i.e., specificity for the HBV pre-S2 antigen.

In this specification, standard single-letter or three-letter abbreviations are used to represent nucleotides and amino acids. The meanings of these abbreviations can be found in standard biochemistry textbooks such as Lehninger, Principles of Biochemistry, 1984, Worth Publishers Inc., New York, pp. 96, 798.

The following examples are intended to further illustrate the present invention without limiting its scope. Unless otherwise specified, percentages given below for solids in solid mixtures, liquids in liquids and solids in liquids are on a wt/wt, vol/vol and wt/vol basis, respectively.

### Example 1: Cloning of the heavy chain gene encoding a murine monoclonal antibody specific for the pre-2 antigen of HBV adr subtype

The chromosomal DNA was isolated from H8 Hybridoma cell line which secreted murine monoclonal antibodies(γ 2b, κ ) specific for the pre-2 antigen of HBV adr subtype(Chung et al., Recent Progress in Molecular Biology and Genetic Engineering in Korea 1, 165-172(1987)) by using Maniatis' method(Maniatis et al., 1989, vide supra).

The isolated DNA was partially digested with restriction endonuclease Sau 3A1, and fractionated by using 10-40% sucrose gradient centrifugation. DNA fragments in the molecular range of 9 to 23kb were collected and cloned into the Bam HI site of λ EMBL3 phage vector(Promega, U.S.A.) to obtain a DNA library(Maniatis et al., 1989, vide supra).

The recombinant phage DNA was packaged in vitro and the recombinant phage so obtained was infected into E. coli KW251 (Promega, U.S.A.); and the transformed E. coli was cultured in LB liquid medium(NaCl 10g, Bacto-trypton 10g, Bacto-yeast extract 5g per ℓ) at 37°C for about 15 minutes. The resulting culture was applied on LB solid medium and incubated at 37°C overnight. As a result, 25,000 plaques were formed per 15-diameter plate containing LB medium.

About 200,000 plaques in the 7 plates were transferred onto nitrocellulose filters, denatured, fixed and subjectd to plaque hybridization(Maniatis et al., 1989, vide supra). As probes for the hybridization, [α-³²P]dATP-nick translated 465bp Pst I fragment which specifies the V region of heavy chain cDNA(pMH)(Hong et al., Cloning and characterization of cDNAs coding for heavy and light chains of a monoclonal antibody specific for the pre-S2 antigen of hepatits B virus, submitted to Gene, 1992), which had been cloned by the present inventors, and [γ-³²P]ATP-kinated synthetic oliconucleotide, 5'-TGTTGAGTTGAGTCAAGATGGCCG-3', modeled on the J-C intron of the heavy chain gene which specifies the intron enhancer of the heavy chain gene were used.

Of about 200,000 plaques, only one plaque positively reacted in both screenings using the above two probes. After amplifying the recombinant phage clone(λ MHG-S2), the insert cloned into the Bam HI site of the λ DNA was isolated, digested with Eco RI; and Southern hybridization using the same two probes as above was carried out (Maniatis et al., 1989, vide supra). As a result, it was confirmed that 6.6kb Eco RI DNA fragment contained the gene encoding V region and the intron enhancer region of the heavy chain gene.

The 6.6 kb-sized Eco RI fragment was subcloned into the Eco RI site of pUC18 to obtain pMHG-S2, whose restriction map was determined as shown in Figure 1A.

The nucleotide sequence of the recombinant plasmid was determined by the dideoxy nucleotide sequencing method using Sequanase(USB Co., U.S.A.) in accordance with the protocol provided by the manufacturer. As a result, it was confirmed that the plasmid DNA comprised a V region exon whose V,D and J regions were functionally rearranged and the intron enhancer of the heavy chain gene; and the nucleotide sequences of the promoter, leader exon, leader intron and V region exon of the heavy chain were determined as shown in Figure 1B. The promoter region comprises a TATA box, an Oct-2(Octamer binding protein) binding site and a heptamer sequence positioned at about 95 bp(-99∼-93bp), about 120bp(-124∼-117bp) and about 140bp(-145∼-139bp), respectively, upstream of the first methionine codon, which are essential for the B-cell specific expression of immunoglobulin genes(Poellinger et al., Mol. Cell. Biol. 9, 747-756(1989); Wirth et al., Nature 329, 174-178 (1987)).

Based on the comparison of the nucleotide sequence encoding the leader in the gene with that of the cloned heavy chain cDNA, it was determined that the gene contained the leader intron having GT and AG as 5'- and 3'-boundary nucleotides, respectively(from 823rd nucleotide to 422nd nucleotide in Figure 1B), separating the leader exon into two parts.

A further comparison of the amino acid sequence deduced from the V region gene of the heavy chain with that of Immunoglobulin of Kabat et al.(Sequences of Proteins of Immunological Interest, 4th Ed. p219, U.S.A.) confirmed that the heavy chain belonged to murine heavy chain subgroup III(C), whose, antigen binding sites, i.e., CDR(complementarity determining regions) 1,2 and 3 were posited at 31st-35th, 49th-68th and 101st-109th amino acids, respectively; and, said CDR 3 contained the diversity region of FL 16 family recombined with the joining region of JH3.

The above structural analyses of the promoter and the V region suggest that the cloned heavy chain gene be functional.

The plasmid pMGH-S2 was deposited with Korean Collection for Type Culture with the accession number of KCTC 0029BP on January 24, 1992 under the Budapest Treaty on the International Recognition of the Deposit of Microorganisms for the Purpose of Patent Procedure.

### Example 2: Cloning of kappa chain gene

The DNA isolated from H8 hybridoma cell line in Example 1 was completely digested with Bam HI, and then electrophoresed on 0.7% agarose gel to separate DNA fragments by their size. The DNA fragments were transferred onto nitrocullulose filters(Millipore, U.S.A.) and subjected to Southern hybridization using[α-³²P]dATP labelled 348-bp DNA fragment of pMK amplified by PCR or 450bp HapI-Eco RI DNA fragment of pMK(see Hong et al., submitted to Gene, 1992, vide supra) which specific for the gene encoding the V region and the C region of the kappa chain, respectively, wherein cDNA encoding kappa chain(pMK) had been cloned from H8 cell line by the present inventors, as a probe(Maniatis et al., 1989). The chromosomal DNA isolated from SP 2/0 cells was also electrophoresed on the same gel and hybridized with the probe. As a result, among the H8 cell line DNA fragments, 6.6kb-size DNA fragment positively reacted with both probes, although the SP 2/0 DNA did not.

The positive DNA fragment was electroeluted from the gel (Maniatis et al, 1989), and then cloned into the Bam HI site of pUC19 to obtain a DNA library. The plasmid DNA library was introduced into E. coli DH1. The bacterial colonies so produced were screened by using colony hybridization method with the above-mentioned probe(Maniatis et al., 1989).

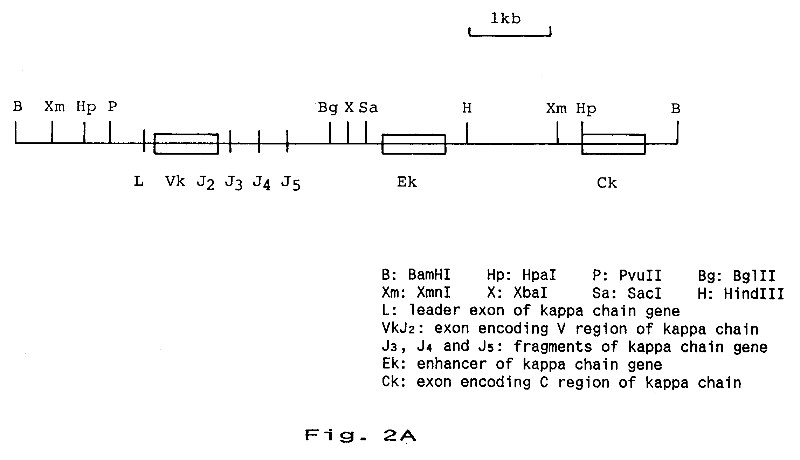

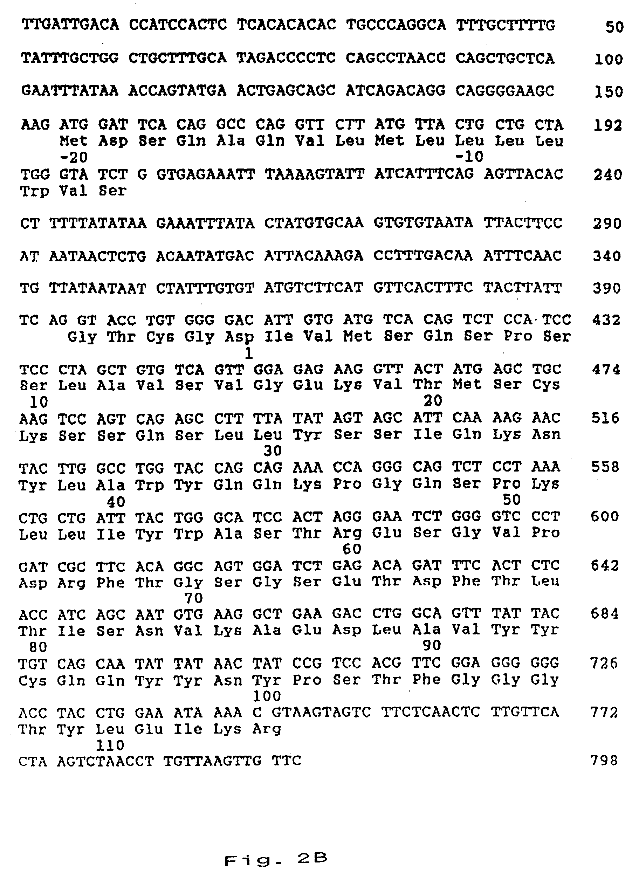

The recombinant plasmid DNA was isolated from each of the several positive bacterial colonies, and, then, its restriction map was determined as shown in Figure 2A, to find that only one clone contained the kappa chain gene. The recombinant DNA was named pMKG-S2, and its nucleotide DNA sequence was determined. The cloned DNA was 6.6kb-sized Bam HI fragment, which contained a functionally rearranged V region exon of the kappa chain gene, the intron containing the enhancer of the kappa chain gene, and a gene encoding of the C region of the kappa chain, Ck exon.

The nucleotide sequences of the promoter, leader exon, leader intron and the V region gene exon of the kappa chain gene were determined as shown in Figure 2B. In the promoter, a TATA box was posited at about 45bp(106th∼111th) upstream from the first methionine codon and an Octamer motif was found at about 85bp(64th∼71st) upstream of the first methionine codon.

Therefore, the kappa chain gene was confirmed to have a kappa chain gene-specific enhancer as well as transcriptional factors-binding sites essential for the B-cell specific expression.

Comparing the amino acid sequence deduced from the kappa chain gene of H8 antibody with that of Kabat et al.(1987, vide supra), it was determined that the kappa chain of H8 antibody belonged to group I of murine kappa chain; and CDR 1,2 and 3 were posited at 24th-40th, 56th-62th and 95th-103th amino acids, respectively. Further, the V region was combined with JK 2 DNA fragment.

The above structural analyses of the promoter and the V region gene suggests that the cloned H8 kappa chain gene be functional.

The plasmid pMKG-S2 was deposited with Korean Collection for Type Culture with the accession number of KCTC 0030BP on January 24, 1992 under the Budapest Treaty on the International Recognition of the Deposit of Microorganisms for the Purpose of Patent Procedure.

### Example 3: Construction of expression vector for producing the heavy chain of chimeric antibody

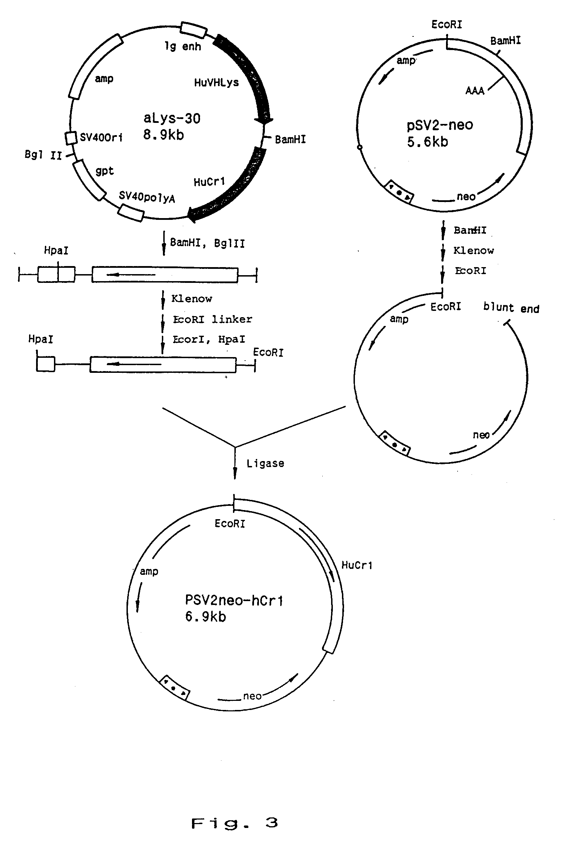

Figure 3 illustrates the strategy for constructing recombinant plasmid SV2neo-hCrl, an intermediate plasmid for constructing the expression vector for producing the heavy chain of chimeric antibody.

The gene encoding the C region of human Immunoglobulin(Ig) heavy chain, Crl, was cloned into the eucaryotic expression vector pSV2-neo as follows: pSV2-neo(Southern et al., J. Mol. Genet. 1, 327-341(1982)) was digested with Bam HI and then treated with Klenow fragment to make the ends of the DNA fragment to be blunt; and the blunt-ended pSV2-neo was further digested with Eco RI.

On the other hand, Bam HI-Bgl II DNA fragment containing the C region gene of the human Ig heavy chain, Crl, which was excised from plasmid aLys-30(MRC, U.K.), was electroeluted and then treated with Klenow fragment to make its ends blunt. The blunt-ended DNA fragment was ligated with Eco RI linker; and then digested with HpaI and Eco RI endonucleases to obtain a 2.2kb-sized HpaI-Eco RI fragment.

The DNA fragment so obtained was ligated to a linearized pSV2-neo vector, which was introduced into E. coli DH1; and a 6.9kb-sized plasmid was isolated therefrom, which was named pSV2neo-hCrl.

The gene encoding the V region of the heavy chain of the murine monoclonal antibody against HBV pre-S2 was cloned into the above pSV2neo-hCrl as follows: The recombinant plasmid pMHG-S2 already prepared to contain said V region gene was digested with Hinc II and then ligated with Eco RI linker; and it was digested with Eco RI to obtain a 2.5kb-sized DNA fragment containing a V region gene.

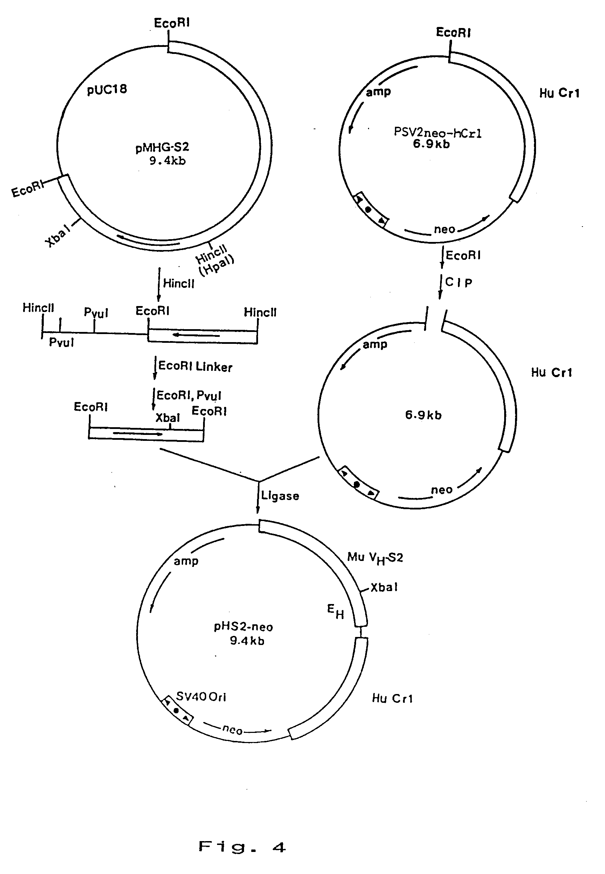

The Eco RI-digested fragment was cloned into the Eco RI site of pSV2neo-hCrl; E. coli DH1 was transformed therewith; and a 9.4kb-sized recombinant plasmid was isolated and named pHS2-neo[see Figure 4].

E. coli DH1 transformed with pHS2-neo(Escherichia coli DH1(pHS2-neo)) was deposited with Korean Collection for Type Culture with the accession number of KCTC 0031BP on January 24, 1992 under the Budapest Treaty on the International Recognition of the Deposit of Microorganisms for the Purpose of Patent Procedure.

Figure 4 illustrates the strategy for constructing the expression vector pHS2-neo for producing the heavy chain of chimeric antibody, starting from pSV2neo-hCrl. In Firgure 4, MuVH-S2 represents the immunoglobulin gene encoding the V region of the heavy chain of the monoclonal antibody isolated from the H8 hybridoma cell line producing the monoclonal antibody against HBV pre-S2; EH represents the intron enhancer of the heavy chain gene; HuCrl represents the C region gene(Crl) of human Ig; SV40 ori and neo represent the replication origin of SV40 and the neomycin-resistant gene, respectively; the arrow represents the direction of transcription; and Eco RI and Xba I respresent the recognition site of the corresponding restriction enzyme.

### Example 4: Construction of expression plasmid for producing the kappa chain of chimeric antibody

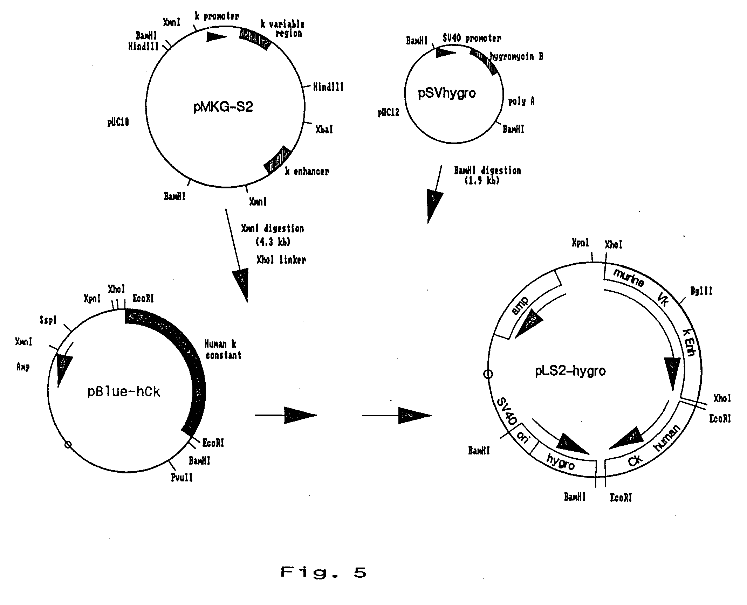

Figure 5 illustrates the strategy for constructing expression plasmid pLS2-hygro for producing the kappa chain of the chimeric antibody against HBV pre-S2.

In order to construct said expression plasmid pLS2-hygro for producing the kappa chain of the chimeric antibody, murine V region gene, human Ck gene and hygromycin B-resistant gene were cloned into pBluescript SK(+)(Stratagene, U.S.A.), an E. coli cloning vector, in accordance with the following steps:
(1) recombinant plasmid pHuCk(Hieter et al., Cell 22, 197-207(1980)) containing the gene(Ck) encoding the C region of the human Ig kappa chain was digested with Eco RI; a 2.6kb-sized DNA fragment was electroeluted; and, then, the fragment was inserted into the Eco RI site of pBluescript SK(+) to obtain a 5.2kb-sized recombinant plasmid pBlue-hCk;
(2) the recombinant plasmid pMKG-S2 obtained in Example 2 was digested with Xmn I; a 4.3kb-sized Xmn I DNA fragment was ligated with Xho I linker and digested with Xho I; and the Xho I fragment was inserted into Xho I site of the pBlue-hCk obtained in step (1) to prepare 9.5kb-sized recombinant plasmid pBlue-hCk-mVk containing the whole chimeric kappa chain gene; and
(3) the expression vector pSV-hygro which has a selective marker for expressing in mammalian cell, was digested with Bam HI; a 1.9kb Bam HI DNA fragment containing the hygromycin-B gene driven by SV40 promoter was electroeluted the fragment; was inserted into the Bam HI site of the recombinant plasmid pBlue-hCk-mVk obtained in step (2) to prepare a 11.4 kb sized recombinant plasmid which was named pLS2-hygro[see Figure 5]; and E. coli DH1 cells were transformed with said pLS2-hygro.

In Figure 5, murine Vk represents the gene encoding the V region of the kappa chain of the monoclonal antibody; KEnh represents the intron enhancer of the kappa chain gene; and Ck human represents the gene encoding the C region of the kappa chain of human immunoglobulin.

Said E. coli DH1 transformed with pLS2-hygro(Escherichia coli DH1(pLS2-hygro)) was deposited with Korean Collection for Type Culture with the accession number of KCTC 0032BP on January 24, 1992 under the Budapest Treaty on the International Recognition of the Deposit of Microorganism for the Purpose of Patent Procedure.

### Example 5: Isolation of transfectoma cell line producing chimeric antibody against HBV pre-S2

A non-Ig secreting murine hybridoma cell line, SP 2/O-Ag 14, was cultured in DMEM medium(Gibco Co.) supplemented with 10% fetal bovine serum(Hyclone Co.) in 7% CO₂; and then transferred into IMEM medium(Gibco Co.).

A transfectoma cell line producing a chimeric antibody against HBV pre-S2 was prepared as follows:
(1) In order to express the chimeric kappa chain gene in the cell line, 10µg of the expression plasmid pLS2-hygro containing the chimeric kappa chain gene was digested with Spe I and Not I to make it linear; the linearized plasmid was added to 0.9mℓ of the IMEM medium containing the cells(2 x 10⁶ cell/mℓ) and introduced to the cells by electroporation method(S · -U · Shin and S. Morrison, Method in Enzymology 32, 1989).
   After 2 days, the IMDM medium containing the cells was supplemeted with 500µg/mℓ hygromycin(Boeringermanhein Co.) and the cells were cultured further for 2 weeks, refreshing the medium at the interval of 3 days. Several tens of cell clones survived were isolated. Each of the clones was cultured, suspended, and then centrifuged. The supernatant was subjected to ELISA(emzyme linked immunosorbent assay).
   That is, 100µℓ of cell extract solution was added to wells of 96 well microtiter plate, incubated at 4°C overnight, blocked with 2% bovine serum albumin, and then washed with TBS-Tween solution(0.05% Tween-20, 10mM Tris-HCl, 0.15M NaCl, pH 7.4). Thereafter, the plate so treated was bound with the anti-human kappa chain antibody conjugated with horseradish peroxidase diluted with 0.3% BSA-containing TBS solution as a secondary antibody.
   The plate was washed with TBS-Tween solution; ABTS^{R} of ELISA Screen Kit(Boeringermanhein Co.) and hydrogen peroxide were added thereto to develop color reaction. The O.D. value at 405nm was determined using micro plate reader(Titertek Multiskan Plus). As a result, about 20 positive clones producing the chimeric kappa chain were selected.
   In order to confirm whether mRNA encoding the chimeric kappa chain was present in cytoplasm of the above positive clones, total RNA was isolated from each clone(Chomezynski and Sacchi, Anal. Biochem. 162, 156-159(1987)); and northern hybrization was carried out with the RNA(Maniatis et al., 1989). As a result, existence of the mRNA transcribed from the gene encoding th kappa chain was proved.
(2) In order to make said clone producing the chimeric kappa chain to express the chimeric heavy chain, simultaneouly, 10µg of pHS2-neo containing the chimeric heavy chain gene was digested with Puv I (whose site was posited in amp gene) for linearization, and then introduced into the cells obtained in step (1) above by employing the electroporation method; and, after 2 days, the transformed cell was cultured in IMEM medium supplemented with 1mg/mℓ of G-418 (Geneticin, Gibco Co.) for 2 weeks, refreshing the medium at the interval of 3 days, to obtain a few survived transfectoma clones.

In order to confirm whether the chimeric heavy chain gene was expressed, 100µl of each of the cultures was subjected to ELISA.

That is, 1µg of HBV pre-S2 peptide(Sigma, Co.) was bound to the surface of wells of a microtiter plate; protein binding sites were blocked with 2% BSA; and then 100µℓ of supernatant of the transfectoma cell cluture was added to the first antibody.

Thereafter, the residual antibody not bound was washed with TBS-Tween solution; anti-human IgG antibody(specific for Fc) conjugated with horseradish peroxidase was added to the wells; and then ABTS^{R} and the substrate H₂O₂ were added thereto to develop color reaction. The O.D. value at 405nm was determined.

As a result, it was confirmed that two transfectoma cell lines, A-44 and 6-31, produced the chimeric antibody against HBV pre-S2[see Figure 6].

Figure 6 shows the results of ELISA of the cell culture of the transfetoma cell line producing the chimeric antibody specific for HBV pre-S2.

As shown in Figure 6, the negative control groups employing murine hybridoma cell line(SP 2/O-Ag 14) culture, 10% FBS containing IMEM medium, or human or murine IgG as the first antibody, respectively, were not colored, while the positive control employing the murine monoclonal antibody against HBV pre-S2 as the first antibody was colored.

In Figure 6, 6-31 and A-44 represent the transfectoma cell lines; A-15 and A-36 represent the cells producing only the kappa chain of the chimeric antibody; hIgG, SP2/O and Med which were used as negative controls represent human immunoglobulin, SP 2/O-Ag 14 cell culture, and medium, respectively; H8 which was used as the positive control represents the H8 hybridoma cell culture; AhIgG and Am IgG which were used as the second antibodies represent the anti-human IgG and the anti-mouse IgG, respectively.

The murine transfectoma A-44 so obtained was deposited with Korean Collectection for Type Culture with the accession number of KCTC 0033BP on January 24, 1992 under the Budapest Treaty on the International Recognition of the Deposit of Microorganisms for the Purpose of Patent Produre.

### Exaple 6: Determination of the level of the chimeric antibody produced from the transfectoma cell line

The level of the chimeric antibody produced by transfectoma cell lines A-44 and 6-31 was determined as follows.

The cell lines producing the murine monoclonal antibody against HBV pre-S2, i.e., A-44, 6-31 and H8 hybridoma cell line, were taken in equal numbers, and cultured for 24 hours, respectively. Each of the cultures was 2-fold diluted and subjected to ELISA by the same procedure as used in Example 5.

As a result, the level of the chimeric antibody produced by the A-44 cell line and 6-31 cell line were about 1.5pg/cell/24 hours and about 1.2pg/cell/24 hours, respectively.

### Example 7: Isolation of the chimeric antibody

The chimeric antibody produced from the transfectoma cell line was isolated as follows.

10⁵ cells/mℓ of the transfectoma cell line was suspension-cultured; the culture was centrifuged at 3,000 rpm for 10 minutes. To the supernatant was added saturated ammonium sulfate solution (pH 7.4) to saturate it to 45% ammonium sulfate and then centrifuged at 10,000 x g to precipitate the antibodies.

The precipitate was dialysed against PBS solution(137mM NaCl, 2.7mM KCl, 4.3mM Na₂PO₄ · 7H₂O, 1.4mM KH₂PO₄) and then loaded in a Protein A-Sepharose 4B column equilibrated with 0.1M sodium phosphate solution(pH 8.0). The chimeric antibody eluted with 0.1M sodium phosphate of pH 6.0 was concentrated by employing Centricon 10(Amicon Co. U.S.A.) and subjected to 10% SDS-PAGE to determine its purity and quantity.

In order to confirm whether the isolated protein was a chimeric antibody, western blotting was carried out(see Towbin et al., Proc. Natl. Acad. Sci. U.S.A. 76, 4350-4354(1979)).

That is, the chimeric antibody on the gel was transferred onto nitrocellulose filters in a transfer buffer solution(24mM Tris, 192mM glycine, 20% methanol, pH 9.3); the filer was blocked with 1% BSA solution; anti-human IgG (Fc)-alkaline phosphatase conjugate (1/1000 fold-diluted solution) was added and stirred gently for 1 hour; and then washed with TBST buffer solution(10mM Tris-HCl, pH 8.0, 100mM NaCl, 0.05% Tween-20).

The filters were colored with alkaline phosphate solution (100mM Tris-HCl, pH 8.0, 10mM NaCl, 5mM MgCl₂) supplemented with NBT(nitroblue tetrazolium chloride) and BCIP(5-bromo-4-chloro-3-indolphosphate p-toluidine salt); and the reaction was stopped with deionized distilled water.

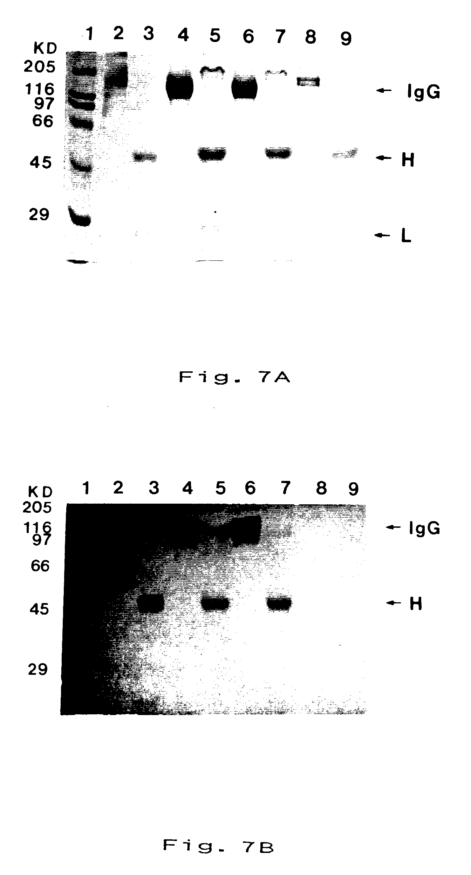

Figures 7A and 7B show 10% SDS-PAGE and the result of western blotting of the chimeric antibody against HBV pre-S2 produced from transfectoma cell lines A-44 and 6-31.

As shown in SDS-PAGE of Figure 7A, a protein band was found to be of 160kd size in non-reduced condition, while 55kd-heavy chain band and 25kd-kappa chain band were found in reduced condition.

As shown in Figure 7B, 160kd of IgG and 55kd of heavy chain bands were reacted with anti-human IgG(Fc)-alkaline phosphatase conjugate. Therefore, it was confirmed that the chimeric antibody was produced from transfectoma cell lines A-44 and 6-31.

While the invention has been described in connection with the above specific embodiments, it should be recognized that various modifications and changes as may be apparent to those skilled in the art to which the invention pertains may be made and also fall within the scope of the invention as defined by the claims that follow.
- All the deposits made at the Korean collection for Type Culture and explicitly mentioned in this application with their respective dates are made under the terms of the Budapest Treaty on the International Recognition of the Deposit of Microorganisms for the Purpose of Patent Procedure.

## Claims

1. A polynucleotide comprising a gene encoding a variable region of an antibody specific for pre-S2 antigen of hepatitis B virus.

2. The polynucleotide of claim 1 comprising a gene encoding a variable region of a heavy chain of the antibody, wherein the nucleotide sequence of the gene is:

ATACACAAAG TTAACTGAGC TGTAAACACA GAAGAGTAAG TGATGATTTC 50

ATTCTATAAT GCTACTTCAG TTTTTATTAT TTCTCAGACA TCTGCACTAG 100

TATTGGAATC CTCTAAGACT TCCATCCCTG AACTCATGAT ATAGCACGAA 150

GACATGCAAA TAATATTTCC CTATGCTCAT AAAAACCAGC CCTGACCATG 200

AAGCTCTAAC AGACACACAA ACCTGGACTC ACAAGTTTTT CTCTTCAGTG 250

ACAGACACAG ACACAGAACA TTCACG ATG TAC TTG GGA CTG AAC 294
Met Tyr Leu Gly Leu Asn
-19
TAT GTA TTC ATA GTT TTT CTC TTA AAT G GTAATTCAT 331
Tyr Val Phe Ile Val Phe Leu Leu Asn
-10
AGATAATAGG GATACTGCAT GTGATTAGAG GGGAGTGAGA GGAACACTGA 381

ATGTTTTGAC TGCTTACTGA CTAGATTCTC TGTGTTTTCA 421

G GT GTC CAG AGT GAA GTG AAG CTT GAG GAG TCT GGA GGA 460
Gly Val Gln Ser Glu Val Lys Leu Glu Glu Ser Gly Gly
1
GGC TTG GTG CAA CCT GGA GGA TCC ATG AAA CTC TCT TGT GCT 502
Gly Leu Val Gln Pro Gly Gly Ser Met Lys Leu Ser Cys Ala
10 20
GCC TCT GGA TTC ACT TTT AGT GAC GCC TGG ATG GAC TGG GTC 544
Ala Ser Gly Phe Thr Phe Ser Asp Ala Trp Met Asp Trp Val
30
CGC CAG TCT CCA GAG AAG GGG CTT GAG TGG GTT GGT GAA ATT 586
Arg Gln Ser Pro Glu Lys Gly Leu Glu Trp Val Gly Glu Ile
40 50
AGA AGC AAA GCT AAT AAT CAT GCA ACA TAC TAT GCT GAG TCT 628
Arg Ser Lys Ala Asn Asn His Ala Thr Tyr Tyr Ala Glu Ser
60
GTG AAA GGG AGG TTC ACC ATC TCA AGA GAT GAT TCC AAA AGT 670
Val Lys Gly Arg Phe Thr Ile Ser Arg Asp Asp Ser Lys Ser
70
AGT GTC TAC CTG CAA ATG AAC AGC TTA AGA GCT GAA GAC ACT 712
Ser Val Tyr Leu Gln Met Asn Ser Leu Arg Ala Glu Asp Thr
80 90
GGC ATT TAT TAC TGT ACC CCC ATT ACT ACG GGC GCC TGG TTT 754
Gly Ile Tyr Tyr Cys Thr Pro Ile Thr Thr Gly Ala Trp Phe
100
GCT TAC TGG GGC CAA GGG ACT CTG GTC ACT GTC TCT GCA 796
Ala Tyr Trp Gly Gln Gly Thr Leu Val Thr Val Ser Ala
110 120
GGTGAGTCCT AACTTC 812

3. The polynucleotide of claim 1 comprising a gene encoding a variable region of a kappa chain of the antibody, wherein the nucleotide sequence of the gene is:

TTGATTGACA CCATCCACTC TCACACACAC TGCCCAGGCA TTTGCTTTTG 50

TATTTGCTGG CTGCTTTGCA TAGACCCCTC CAGCCTAACC CAGCTGCTCA 100

GAATTTATAA ACCAGTATGA ACTGAGCAGC ATCAGACAGG CAGGGGAAGC 150

AAG ATG GAT TCA CAG GCC CAG GTT CTT ATG TTA CTG CTG CTA 192
Met Asp Ser Gln Ala Gln Val Leu Met Leu Leu Leu Leu
-20 -10
TGG GTA TCT G GTGAGAAATT TAAAAGTATT ATCATTTCAG AGTTACAC 240
Trp Val Ser

CT TTTTATATAA GAAATTTATA CTATGTGCAA GTGTGTAATA TTACTTCC 290

AT AATAACTCTG ACAATATGAC ATTACAAAGA CCTTTGACAA ATTTCAAC 340

TG TTATAATAAT CTATTTGTGT ATGTCTTCAT GTTCACTTTC TACTTATT 390

TC AG GT ACC TGT GGG GAC ATT GTG ATG TCA CAG TCT CCA TCC 432
Gly Thr Cys Gly Asp Ile Val Met Ser Gln Ser Pro Ser
1
TCC CTA GCT GTG TCA GTT GGA GAG AAG GTT ACT ATG AGC TGC 474
Ser Leu Ala Val Ser Val Gly Glu Lys Val Thr Met Ser Cys
10 20
AAG TCC AGT CAG AGC CTT TTA TAT AGT AGC ATT CAA AAG AAC 516
Lys Ser Ser Gln Ser Leu Leu Tyr Ser Ser Ile Gln Lys Asn
30
TAC TTG GCC TGG TAC CAG CAG AAA CCA GGG CAG TCT CCT AAA 558
Tyr Leu Ala Trp Tyr Gln Gln Lys Pro Gly Gln Ser Pro Lys
40 50
CTG CTG ATT TAC TGG GCA TCC ACT AGG GAA TCT GGG GTC CCT 600
Leu Leu Ile Tyr Trp Ala Ser Thr Arg Glu Ser Gly Val Pro
60
GAT CGC TTC ACA GGC AGT GGA TCT GAG ACA GAT TTC ACT CTC 642
Asp Arg Phe Thr Gly Ser Gly Ser Glu Thr Asp Phe Thr Leu
70
ACC ATC AGC AAT GTG AAG GCT GAA GAC CTG GCA GTT TAT TAC 684
Thr Ile Ser Asn Val Lys Ala Glu Asp Leu Ala Val Tyr Tyr
80 90
TGT CAG CAA TAT TAT AAC TAT CCG TCC ACG TTC GGA GGG GGG 726
Cys Gln Gln Tyr Tyr Asn Tyr Pro Ser Thr Phe Gly Gly Gly
100
ACC TAC CTG GAA ATA AAA C GTAAGTAGTC TTCTCAACTC TTGTTCA 772
Thr Tyr Leu Glu Ile Lys Arg
110
CTA AGTCTAACCT TGTTAAGTTG TTC 798

4. A recombinant plasmid comprising a gene encoding a variable region of an antibody specific for pre-S2 antigen of hepatitis B virus.

5. The recombinant plasmid of claim 4 which is pMHG-S2 or pMKG-S2.

6. A host cell transformed with the plasmid of claim 4.

7. A recombinant DNA comprising a gene encoding a heavy chain variable region of a murine monoclonal antibody specific for HBV pre-S2 antigen and a gene encoding a heavy chain constant region of human immunoglobulin.

8. An expression vector comprising the recombinant DNA of claim 7, wherein the recombinant DNA is positioned at a site capable of being expressed in a compatible host cell.

9. The expression vector of claim 8 which is pHS2-neo.

10. A recombinant DNA comprising a gene encoding a kappa chain variable region of a murine monoclonal antibody specific for HBV pre-S2 antigen and a gene encoding a kappa chain constant region of human immunoglobulin.

11. An expression vector comprising the recombinant DNA of claim 10, wherein the recombinant DNA is positioned at a site capable of being expressed in a compatible host cell.

12. The expression vector of claim 11 which is pLS2-hygro.

13. A host cell transformed with the expression vector of claim 8 or 11.

14. The host cell of claim 13 which is E. coli DH1 (pHS2-neo) or E. coli DH1(pLS2-hygro).

15. A transfectoma cell line, which is co-transfected with the expression vectors of claims 8 and 11.

16. The transfectoma cell line of claim 15, which is cotransfected with pHS2-neo and pLS2-hygro.

17. A murine/human chimeric antibody speicific for HBV pre-S2 antigen.

18. The murine/human chimeric antibody of claim 17, which is produced from the transfectoma cell line of claim 15.

19. A method for preparing the murine/human chimeric antibody of claim 17, which comprises: culturing the transfectoma cell line of claim 15 and isolating the antibody therefrom.
